(19) Europäisches Patentamt
European Patent Office
Office européen des brevets

(11) Veröffentlichungsnummer: **0 024 334**
**B1**

(12) # EUROPÄISCHE PATENTSCHRIFT

(45) Veröffentlichungstag der Patentschrift: **20.07.83**

(51) Int. Cl.³: **C 07 D 265/30,** A 61 K 31/535

(21) Anmeldenummer: **80104656.6**

(22) Anmeldetag: **07.08.80**

(54) **Neue Morpholinderivate, Verfahren zu deren Herstellung, diese Derivate enthaltende pharmazeutische Präparate.**

(30) Priorität: **17.08.79 CH 7560/79**
**29.05.80 CH 4187/80**

(43) Veröffentlichungstag der Anmeldung:
**04.03.81 Patentblatt 81/9**

(45) Bekanntmachung des Hinweises auf die Patenterteilung:
**20.07.83 Patentblatt 83/29**

(84) Benannte Vertragsstaaten:
**AT BE CH DE FR GB IT LI LU NL SE**

(56) Entgegenhaltungen:
**EP-A-0 005 541**
**EP-A-0 007 093**
**EP-A-0 007 479**
**EP-A-0 008 686**
**EP-A-0 014 999**
**DE-A-2 656 747**
**DE-A-2 752 096**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Pfiffner, Albert, Dr., Grampenweg 10, CH-8180 Bülach (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

**EP 0 024 334 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue Morpholinderivate, Verfahren zu deren Herstellung, diese Derivate enthaltende pharmazeutische Präparate

Die Erfindung betrifft heterocyclische Verbindungen der allgemeinen Formel

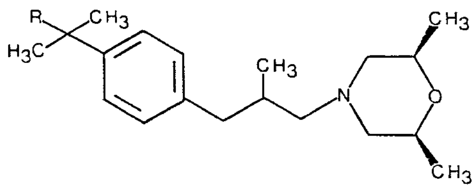
I

worin R Äthyl oder Phenyl bedeutet,
und Salze sowie N-Oxyde dieser Verbindungen.

Die Erfindung betrifft ferner ein Verfahren zur Herstellung von Verbindungen der Formel I und von deren Salzen und N-Oxyden, fungizide Mittel, welche diese Verbindungen enthalten, Verfahren zur Herstellung dieser Mittel sowie die Verwendung dieser Mittel.

Aus der DE-A-2 752 096 sind Verbindungen bekannt, von welchen sich die erfindungsgemässen Verbindungen der Formel I dadurch unterscheiden, dass sie cis-Isomere darstellen.

Das Verfahren zur Herstellung der erfindungsgemässen Verbindungen ist dadurch gekennzeichnet, dass man

a) ein Halogenid der Formel

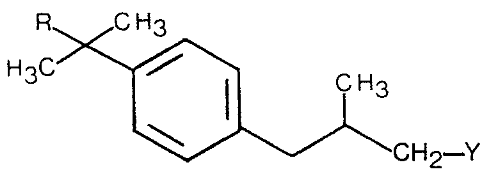
II

worin R die oben angegebene Bedeutung besitzt und Y Chlor, Brom oder Jod bedeutet,
mit einer Verbindung der Formel

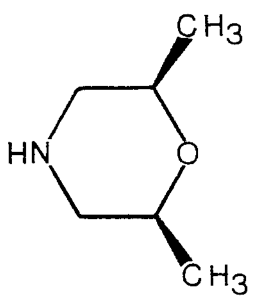
III

umsetzt, oder

b) eine Verbindung der Formel

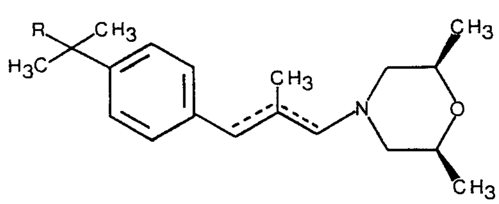
IV

worin R die oben angegebene Bedeutung besitzt und eine der beiden gestrichelten Linien eine weitere Bindung darstellt,
reduziert, oder

c) eine Verbindung der allgemeinen Formel

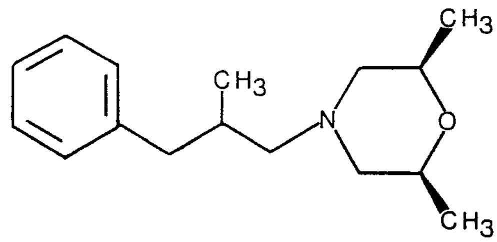
V

mit einer Verbindung umsetzt, die ein Carboniumion der allgemeinen Formel

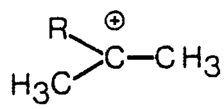
VI

worin R die oben angegebene Bedeutung besitzt, liefert, oder

d) eine Verbindung der Formel

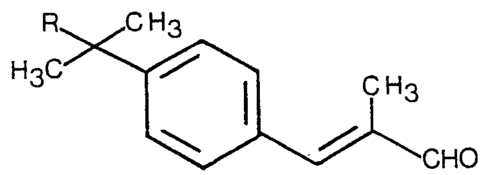
VII

worin R die obige Bedeutung hat,
unter hydrierenden Bedingungen mit einer Verbindung der Formel III umsetzt, oder dass man, zwecks Herstellung eines N-Oxyds, eine Verbindung der Formel I mit Wasserstoffperoxid oder Persäuren behandelt, oder dass man, zwecks Herstellung eines Salzes, eine Base der Formel I mit einer Säure in an sich bekannter Weise in ein Salz überführt.

Gemäss Verfahrensvariante a) wird ein Halogenid der Formel II mit einem Amin der Formel III, zweckmässig in einem inerten Lösungsmittel, beispielsweise in einem Äther wie Diäthyläther, Tetrahydrofuran oder Dioxan, oder in Dimethylsulfoxid, vorzugsweise in einem höhersiedenden Alkohol, wie Äthylenglykol oder Glycerin, in Gegenwart einer Base, wie beispielsweise Triäthylamin oder einem Überschuss an Amin der Formel III umgesetzt. Das Gemisch wird vorzugsweise in einem Temperaturbereich zwischen 50 und 150°C zur Reaktion gebracht. Besonders bevorzugt ist als Lösungsmittel Äthylenglykol und eine Temperatur von 100-110°C.

Gemäss Verfahrensvariante b) wird eine Verbindung der Formel IV reduziert. Wenn als Ausgangsmaterial der Formel IV eine Verbindung verwendet wird, worin sich die Doppelbindung in α-Stellung zum Morpholinrest befindet, so kann die Reduktion katalytisch oder mit Ameisensäure erfolgen.

Als Katalysatoren eignen sich besonders Edelmetallkatalysatoren wie beispielsweise Platin, Palladium — gegebenenfalls auf Kohle niedergeschlagen — sowie Raney-Nickel. Bevorzugt ist Palladium auf Kohle. Für die katalytische Reduktion geeignete Lösungsmittel sind Kohlenwasserstoffe wie Benzol,

Toluol oder Xylol, sowie Alkohole wie Methanol oder Äthanol. Bevorzugt ist Toluol. Als Reaktionstemperatur wird vorteilhaft ein Intervall zwischen 0 und 50°C, bevorzugt Raumtemperatur gewählt. Die Reduktion des Enamins mit Ameisensäure wird vorzugsweise in Abwesenheit eines Lösungsmittels durchgeführt, zum Enamin wird bei einer Temperatur von 0-100°C, vorzugsweise 50-70°C, die Ameisensäure, notwendigenfalls unter Kühlung, zugetropft.

Bei Verwendung eines Ausgangsmaterials der Formel IV, worin sich die Doppelbindung in α-Stellung zum Phenylrest befindet, kann die Reduktion katalytisch erfolgen. Als Katalysator dient hierbei vorzugsweise Platin oder Palladium, wobei als Lösungsmittel Wasser oder Alkohol zur Verwendung gelangt. Um eine mögliche Hydrogenolyse zu vermeiden, wird dem Reaktionsgemisch mindestens ein Äquivalent Säure, vorzugsweise Salzsäure, zugesetzt.

Die Alkylierung der Verbindung der allgemeinen Formel V gemäss Verfahrensvariante c) erfolgt in Anwesenheit einer geeigneten Menge eines Friedel-Crafts-Katalysators. Als Katalysator eignen sich die bekannten Friedel-Crafts-Katalysatoren wie z.B. Aluminiumchlorid, Eisenchlorid, Zinkchlorid, Bortrifluorid, Zinnchlorid, Fluorwasserstoff, Schwefelsäure und Phosphorsäure. Für die Zwecke der vorliegenden Erfindung ist Schwefelsäure besonders bevorzugt.

Die Verwendung eines inerten organischen Lösungsmittels ist hierbei nicht zwingend, wird jedoch bevorzugt. Als inerte organische Lösungsmittel eignen sich besonders Alkane wie Hexan, Cyclohexan, chlorierte Kohlenwasserstoffe wie Chloroform, Äthylendichlorid und Methylenchlorid, wobei Methylenchlorid besonders bevorzugt wird. Die Temperatur der Alkylierungsreaktion ist nicht kritisch, liegt jedoch in der Regel zwischen 0-50°C, vorzugsweise zwischen 18-20°C.

Vorzugsweise und insbesondere im Falle der Verwendung von Schwefelsäure wird nach Beendigung der Reaktion das erhaltene Produkt in der Salzform mit einem inerten organischen Lösungsmittel wie z.B. Methylenchlorid extrahiert. Falls erwünscht kann das freie Amin mit einer geeigneten Base wie Natronlauge, Kalilauge, Soda, Pottasche oder Calciumhydroxid erhalten werden.

Bevorzugte Verbindungen, welche die Carboniumionen der allgemeinen Formel VI liefern, sind die entsprechenden tert. Alkohole, wie 2-Methyl-2-butanol oder tert. Chloride, wie 2-Chlor-2-methyl-butan.

Die Umsetzung des substituierten Zimtaldehyds der Formel VII mit dem cis-2,6-Dimethylmorpholin der Formel III erfolgt unter hydrierenden Bedingungen, beispielsweise unter Verwendung eines Palladiumkatalysators, z.B. in Methanol.

Zwecks Herstellung der N-Oxyde der Verbindungen der Formel I wird eine Verbindung der Formel I mit Wasserstoffperoxyd oder einer Persäure behandelt. Als Lösungsmittel dienen, im Falle der Verwendung von Wasserstoffperoxyd als Oxydationsmittel, Alkohole wie Methanol, Äthanol oder Isopropanol, wobei letzterer bevorzugt ist. Bevorzugte Reaktionstemperaturen liegen zwischen 0 und 50°C, besonders bevorzugt bei 40°C. Als Persäuren können beispielsweise Peressigsäure, Perbenzoesäure, Metachlorperbenzoesäure, Peradipinsäure verwendet werden. Als Lösungsmittel für die Persäuren dienen vorzugsweise halogenierte Kohlenwasserstoffe wie Methylenchlorid, Chloroform oder Äthylenchlorid. Als Reaktionstemperaturen eigenen sich die gleichen wie vorstehend für die Reaktion mit Wasserstoffperoxyd beschrieben.

Verbindungen der Formel I bilden Salze mit organischen und anorganischen Säuren. Als Salze für die Verbindungen der Formel I kommen insbesondere Salze mit physiologisch verträglichen Säuren in Frage. Hierzu gehören vorzugsweise die Halogenwasserstoffsäuren, wie z.B. die Chlorwasserstoffsäure und die Bromwasserstoffsäure, ferner Phosphorsäure, Salpetersäure, ausserdem mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, und schliesslich Sulfonsäuren, wie die 1,5-Naphthalin-disulfonsäure. Die Herstellung von derartigen Salzen erfolgt in an sich bekannter Weise.

Die Ausgangsmaterialien der Formeln II und VI sind bekannte Verbindungen. Die Ausgangsmaterialien III und IV sind als cis/trans-Gemische bekannt. Die cis-Ausgangsmaterialien III und IV sind analog den Verfahren für die Herstellung der cis/trans-Gemische erhältlich.

Die Ausgangsmaterialien der Formel IV, worin sich die Doppelbindung in α-Stellung zum Morpholinrest befindet, können beispielsweise erhalten werden durch Umsetzung eines entsprechend substituierten Phenyl-2-methyl-propionaldehyds mit cis-2,6-Dimethylmorpholin. Die Hydrierung der so erhaltenen Verbindungen der Formel IV erfolgt zweckmässigerweise in situ.

Die Ausgangsmaterialien der Formel IV, worin sich die Doppelbindung in α-Stellung zum Phenylrest befindet, können beispielsweise durch Umsetzung eines Halogenids, welches der Formel II entspricht, welches jedoch in α-Stellung zum Phenylrest eine Doppelbindung aufweist, mit cis-2,6-Dimethylmorpholin erhalten werden.

Die Verbindungen der allgemeinen Formel V können hergestellt werden, indem man eine Verbindung der allgemeinen Formel

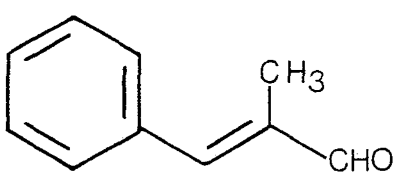

VIII

mit einer Verbindung der allgemeinen Formel III vermischt und katalytisch hydriert.

Als Lösungsmittel wird vorzugsweise ein organisches Lösungsmittel z.B. ein Alkohol, wie Methanol verwendet. Die Temperatur ist nicht kritisch, liegt jedoch in der Regel zwischen 0-50°C.

Bei der katalytischen Hydrierung dienen die üblichen Hydrierungskatalysatoren wie z.B. Platin, Raney-Nickel oder Palladium, wobei 5% Palladiumkohle besonders bevorzugt wird.

Die erfindungsgemässen Verbindungen enthalten in der den Morpholinrest mit dem p-substituierten

Phenylrest verbindenden Propylenkette ein asymmetrisches C-Atom und können daher in Form der Racemate und in Form der optischem Antipoden auftreten. Letztere können aus den anfallenden Racematen durch Spaltung mit optisch aktiven Säuren erhalten werden, z.B. mit (+)-Camphersäure, (+)-Campher-10-sulfonsäure, O,O'-Dibenzoylweinsäure (L- oder D-Form), L(+)-Weinsäure, D(—)-Weinsäure, L(+)-Glutaminsäure-4-sulfonat, L(—)-Äpfelsäure oder D(+)-Äpfelsäure. Diese Spaltung kann nach üblichen Spaltungsmethoden erfolgen.

Die erfindungsgemässen Verbindungen besitzen fungizide Wirkung und können dementsprechend zur Bekämpfung von Fungi in der Landwirtschaft und im Gartenbau Verwendung finden. Die Verbindungen eignen sich besonders zur Bekämpfung von echten Mehltaupilzen wie beispielsweise Erysiphe graminis (Getreidemehltau), Erysiphe cichoracearum (Gurkenmehltau), Podosphaera leucotricha (Apfelmehltau), Sphaerotheca pannosa (Rosenmehltau), Oidium tuckeri (echter Rebmehltau); Rostkrankheiten wie beispielsweise solche der Gattungen Puccinia, Uromyces und Hemileia, insbesondere Puccinia graminis (Getreideschwarzrost), Puccinia coronata (Haferkronenrost), Puccinia sorghi (Maisrost), Puccinia striiformia (Getreidegelbrost), Puccinia recondita (Getreidebraunrost), Uromyces fabae und appendiculatus (Buschbohnenroste) sowie gegen Hemileia vastatrix (Kaffeerost) und Phragmidium mucronatum (Rosenrost).

Ferner wirken diese Verbindungen auch gegen folgende phytopathogenen Pilze:

Ustilago avenae (Flugbrand), Venturia inaequalis (Apfelschorf), Cercospora arachidicola (Erdnuss-Blattfleckenkrankheit), Ophiobolus graminis (Getreide-Fusskrankheit), Septoria nodorum (Getreideblatt- und Spelzbräune) oder Marssonina rosae (Rosen-Sternrusstau). Einzelne Substanzen aus dieser Verbindungsklasse besitzen ausgeprägte Nebenwirkungen gegen verschiedene Species folgender Gattungen: Rhizoctonia, Tilletia, Helminthosporium sowie auch teilweise gegen Peronospora, Coniophora, Lenzites, Corticium, Thielaviopsis und Fusarium.

Ausserdem wirken Verbindungen der Formel I auch gegen phytopathogene Bakterien wie beispielsweise Xanthomonas vesicatoria, Xanthomonas oryzae und andere Xanthomonaden sowie auch gegen verschiedene Arten von Erwinia, z.B. Erwinia tracheiphila.

Vor allem aber sind die erfindungsgemässen Wirkstoffe aufgrund ihrer fungistatischen und fungiziden Wirkung geeignet zur Bekämpfung von Infektionen, die durch Pilze und Hefen hervorgerufen werden, beispielsweise der Genera Candida, Trichophyten oder Histoplasma. Sie sind insbesondere wirksam gegen Candida-Arten wie Candida albicans und eignen sich vorzugsweise zur lokalen Therapie oberflächlicher Infektionen der Haut und der Schleimhäute, insbesondere des Genitaltraktes, beispielsweise Vaginitis, speziell verursacht durch Candida. Die Applikationsform der Wahl ist die lokale, so dass die Wirkstoffe als therapeutisch aktive Präparate in Form von Salben, Zäpfchen, Suppositorien, Ovula oder anderen geeigneten Formen zur Anwendung kommen können.

Die Herstellung der pharmazeutischen Präparate kann in an sich bekannter Weise durch Vermischen der Wirkstoffe mit üblichen organischen oder anorganischen inerten Trägermaterialien und/oder Hilfsstoffen, wie Wasser, Gelatine, Milchzucker, Stärke, Magnesiumstearat, Talk, pflanzliche Öle, Polyalkylenglykole, Vaseline, Konservierungs-, Stabilisierungs-, Netz- oder Emulgiermittel, Salze zur Veränderung des osmotischen Druckes oder Puffer, erfolgen.

Die Dosierung erfolgt nach individuellen Erfordernissen, jedoch dürfte eine Applikation von täglich 1-2 Tabletten, die 50-100 mg Wirkstoff enthalten, während weniger Tage eine bevorzugte Dosierung darstellen. Die Salben enthalten zweckmässigerweise 0,3-5%, vorzugsweise 0,5-2%, besonders bevorzugt 0,5-1% an Wirkstoff. Der nachstehende Versuchsbericht und die in der Tabelle angegebenen Resultate geben dem Fachmann ebenfalls die notwendige Information für die Dosierung der Wirkstoffe.

Die erfindungsgemässen Verbindungen wurden auf ihre Wirksamkeit gegen Candida albicans in dem in Path. Microbiol. 23: 62-68 (1960) beschriebenen Vaginal-Candidiasis-Test an der Ratte geprüft, wobei die folgenden Resultate erhalten wurden:

| Verbindung | Konzentration in % bei der eine «ED 50» Wirkung eintrat |
|---|---|
| cis-4-[3-(p-tert.-Amyl-phenyl)-2-methyphenyl]-2,6-dimethyl-morpholin | 0,01 |
| cis-4-/3-[p-(α,α-Dimethyl-benzyl)-phenyl]-2-methyl-propyl/-2,6-dimethyl-morpholin | 0,01 |

*Beispiel 1*

230 g 3-(p-tert.-Amyl-phenyl)-2-methyl-propionaldehyd und 137 g cis-2,6-Dimethyl-morpholin werden in 1000 ml Toluol in einem Wasserabscheider unter Stickstoffbegasung 16 Stunden bis zur Beendigung der Wasserabspaltung am Rückfluss erhitzt. Bei Raumtemperatur werden unter Stickstoffbegasung 17,5 g 5% Palladium auf Kohle zugegeben und anschliessend bis zur Beendigung der Wasserstoffaufnahme hydriert, vom Katalysator abfiltriert und das Toluol am Vakuum abgedampft. Durch Destillation des Rückstandes wird reines cis-4-[3-(p-tert.-Amyl-phenyl)-2-methyl-propyl]-2,6-dimethyl-morpholin vom Siedepunkt 120°C/0,1 Torr erhalten.

*Beispiel 2*

In zu Beispiel 1 analoger Weise erhält man durch Umsetzung von 3-[p-(α,α-Dimethyl-benzyl)-phenyl]-2-methyl-propionaldehyd mit cis-2,6-Dimethyl-morpholin und Hydrierung des cis-4-/3-[p-(α,α-Dimethyl-benzyl)-phenyl]-2-methyl-propyl/-2,6-dimethyl-morpholin mit einem Siedepunkt von 162°C/0,04 Torr.

*Beispiel 3*

Zu 1223 g cis-4-(2-Methyl-3-phenyl-propyl)-2,6--dimethyl-morpholin in 4 l Methylenchlorid werden bei −5°C innerhalb 45 Minuten 4941 g konz. Schwefelsäure und anschliessend innerhalb 60 Minuten 520 g 2-Methyl-2-butanol getropft. Die Reaktionslösung wird unter Eiskühlung mit Wasser versetzt und die wässerige Phase mehrmals mit Methylenchlorid extrahiert. Die organischen Extrakte werden mit Natronlauge und anschliessend mit Wasser gewaschen, getrocknet, eingedampft und das ölige cis-4-[3-(p-tert.-Amyl-phenyl)-2-methylpropyl]-2,6--dimethyl-morpholin im Vakuum destilliert, Siedepunkt 120°C/0,1 Torr.

Das hierbei als Ausgangsprodukt verwendete cis--4-(2-Methyl-3-phenyl-propyl)-2,6-dimethyl-morpholin kann wie folgt erhalten werden:

1000 g α-Methyl-zimtaldehyd, 10 l Methanol und 789 g cis-2,6-Dimethyl-morpholin werden unter einer Stickstoffatmosphäre mit 50 g 5%iger Palladium-Kohle versetzt. Anschliessend wird unter Wasserkühlung bei 30°C bis zur Beendigung der Wasserstoffaufnahme hydriert, vom Katalysator abfiltriert, das Methanol unter vermindertem Druck abdestilliert und dann das rohe cis-4-(2-Methyl-3-phenyl-propyl)--2,6-dimethyl-morpholin destilliert. Das 99% reine Produkt siedet bei 109°C/0,055 Torr.

*Beispiel 4*

20,0 g p-(α,α-Dimethyl-benzyl)-α'-methyl-zimtaldehyd und 9,6 g cis-2,6-Dimethyl-morpholin werden in 60 ml Methanol unter Argonbegasung mit 1 g 5% Palladium auf Kohle versetzt und anschliessend bis zur Beendigung der Wasserstoffaufnahme hydriert. Die vom Katalysator befreite Reaktionslösung wird unter vermindertem Druck eingedampft, mit Chloroform an Aluminiumoxyd (Aktivitätsstufe II) chromatographiert und anschliessend im Hochvakuum destilliert. Man erhält cis-4-/3-[p-(α,α-Dimethyl-benzyl)-phenyl]-2-methyl-propyl/-2,6-dimethyl-morpholin mit einem Siedepunkt von 162°C/0,04 Torr.

*Beispiel 5*

In Analogie zu den Angaben in Beispiel 4 erhält man, ausgehend von p-(tert.-Amyl-phenyl)-α'-methyl-zimtaldehyd und cis-2,6-Dimethyl-morpholin, das cis-4-[3-(p-tert.-Amyl-phenyl)-2-methyl-propyl]-2,6-dimethyl-morpholin.

*Beispiel 6*

Zu einer Lösung von 22,7 g 2,6-cis-Dimethyl-morpholin in 70 ml Äthylenglykol werden bei 125°C langsam 44,3 g 3-(p-tert.-Amyl-phenyl)-2-methyl--propylbromid zugetropft und 48 Stunden bei dieser Temperatur gerührt. Nach dem Abkühlen wird mit 2n Salzsäure versetzt und es werden mit Äther die Neutralteile extrahiert. Anschliessend wird die salzsaure Lösung mit 5n Natronlaugelösung alkalisch gestellt, mit Äther extrahiert, der Ätherextrakt mit Wasser gewaschen, über Natriumsulfat getrocknet und eingedampft. Durch Destillation wird reines cis-4-[3-(p--tert.-Amyl-phenyl)-2-methyl-propyl]-2,6-dimethyl--morpholin, Siedepunkt 120°C/0,1 Torr, erhalten.

*Beispiel 7*

Zu einer Lösung von 22,7 g 2,6-cis-Dimethyl-morpholin in 80 ml Äthylenglykol werden bei 125°C langsam 51,8 g 3-[p-(α,α-Dimethyl-benzyl)-phenyl]-2-methyl-propylbromid zugetropft und 48 Stunden bei dieser Temperatur gerührt. Die Aufarbeitung erfolgt in Analogie zu Beispiel 6. Durch Destillation wird reines cis-4-/3-[p-(α,α-Dimethyl-benzyl)-phenyl]-2-methyl-propyl/-2,6-dimethyl-morpholin, Siedepunkt 162°C/0,04 Torr, erhalten.

*Beispiel 8*

266 g cis-4-[3-(p-tert.-Amyl-phenyl)-2-methyl--propyl]-2,6-dimethyl-morpholin werden in 500 ml absolutem Äthanol gelöst und bei Raumtemperatur 500 ml einer 28%-Chlorwasserstoff-Äthanollösung zugetropft. Die homogene Lösung wird am Rotationsverdampfer zur Trockne eingeengt und der Rückstand aus 100 ml Wasser umkristallisiert. Das Kristallisat wird mit Wasser gewaschen und im Vakuumtrockenschrank bei 55°C getrocknet. Man erhält das Hydrochlorid von cis-4-[3-(p-tert.-Amyl--phenyl)-2-methyl-propyl]-2,6-dimethyl-morpholin als weisse, leicht hygroskopische Kristalle mit Smp. 204-206°C.

*Beispiel 9*

Zu 21 g cis-4-[3-(p-tert.-Amyl-phenyl)-2-methyl--propyl]-2,6-dimethyl-morpholin wird unter Aceton-Trockeneiskühlung eine Lösung von 60 ml 30%-Wasserstoffperoxid in 60 ml Eissigsäureanhydrid so zugetropft, dass die Reaktionstemperatur 50°C nicht übersteigt und anschliessend 16 Stunden bei Raumtemperatur weiterreagieren gelassen. Der Ansatz wird mittels Dünnschichtchromatographie (Hexan-Äther 1:1) überprüft. Zwecks Zerstörung des überschüssigen Peroxids wird die Reaktionslösung auf −10°C abgekühlt und mit 200 ml 40%-Kalilauge versetzt. Nach 20stündigem Nachrühren wird mit 500 ml Wasser verdünnt und mit Chloroform erschöpfend extrahiert. Die vereinigten Chloroformextrakte werden neutralgewaschen, getrocknet und eingedampft. Durch Umkristallisation des Rückstandes aus 100 ml Pentan wird reines cis-4-[3-(p-tert.--Amyl-phenyl)-2-methyl-propyl]-2,6-dimethyl-morpholin-4-oxid erhalten.

Die folgenden Beispiele beschreiben die Herstellung pharmazeutischer Präparate:

*Beispiel 10*

Es werden in üblicher Weise Vaginaltabletten folgender Zusammensetzung hergestellt:

| | |
|---|---|
| cis-4-[3-(p-tert.-Amyl-phenyl)-2-methyl--propyl]-2,6-dimethyl-morpholin | 50 mg |
| Sek. Calciumphosphat $2H_2O$ | 400 mg |
| Direkt pressbare Stärke STA-RX1500 | 261 mg |
| Milchzucker (sprühgetrocknet) | 100 mg |
| Polyvinylpyrrolidon | 25 mg |
| Zitronensäure | 5 mg |
| Magnesiumstearat | 6 mg |
| | 847 mg |

*Beispiel 11*

Es wird eine Salbe folgender Zusammensetzung hergestellt:

| | |
|---|---|
| cis-4-/3-[p-(α,α-Dimethyl-benzyl)-phenyl]-2-methyl-propyl/-2,6-dimethyl-morpholin | 0,7 g |
| Cetylalkohol | 3,6 g |
| Wollfett | 9,0 g |
| Vaseline, weiss | 79,3 g |
| Paraffinöl | 7,4 g |
| | 100,0 g |

*Beispiel 12*

Es wird eine Creme folgender Zusammensetzung hergestellt:

| | | |
|---|---|---|
| cis-4-[3-(p-tert.-Amyl-phenyl)-2-methyl-propyl]-2,6-dimethyl-morpholin | | 0,7 g |
| Polyoxyäthylenstearat | | 3,3 g |
| Stearylalkohol | | 8,0 g |
| Dickflüssiges Paraffinöl | | 10,0 g |
| Vaseline, weiss | | 10,0 g |
| Carboxyvinylpolymer CARBOPOL 934 Ph | | 0,3 g |
| NaOH, reinst | | 0,07 g |
| Wasser, entsalzt | ad | 100,0 g |

**Patentansprüche für die Vertragsstaaten:**
BE, CH, DE, FR, GB, IT, LI, LU, NL, SE

1. Verbindungen der allgemeinen Formel

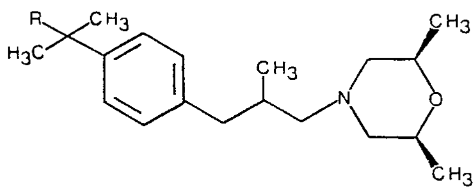

I

worin R Äthyl oder Phenyl bedeutet, und Salze sowie N-Oxyde dieser Verbindungen.

2. cis-4-[3-(p-tert.-Amyl-phenyl)-2-methyl-propyl]-2,6-dimethyl-morpholin und dessen Salze sowie dessen N-Oxyd.

3. cis-4-/3-[p-(α,α-Dimethyl-benzyl)-phenyl-2-methyl-propyl/-2,6-dimethyl-morpholin und dessen Salze, sowie dessen N-Oxyd.

4. Verbindungen nach einem der Ansprüche 1 bis 3 als Antimykotika.

5. Verfahren zur Herstellung von Verbindungen der allgemeinen Formel

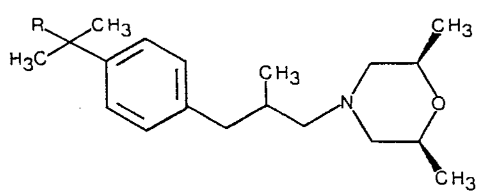

I

worin R Äthyl oder Phenyl bedeutet, und von Salzen und N-Oxyden hiervon, dadurch gekennzeichnet, dass man

a) ein Halogenid der Formel

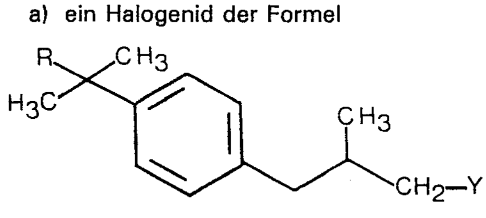

II

worin R die oben angegebene Bedeutung besitzt und Y Chlor, Brom oder Jod bedeutet, mit einer Verbindung der Formel

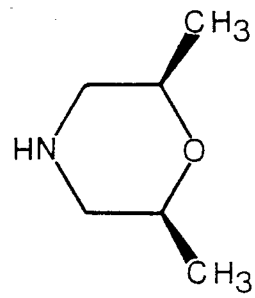

III

umsetzt, oder

b) eine Verbindung der Formel

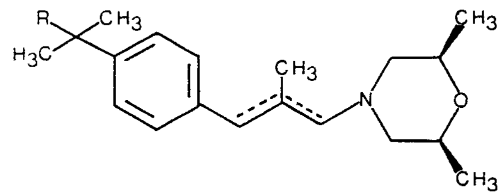

IV

worin R die obige Bedeutung hat und eine der beiden gestrichelten Linien eine weitere Bindung darstellt, reduziert, oder

c) eine Verbindung der allgemeinen Formel

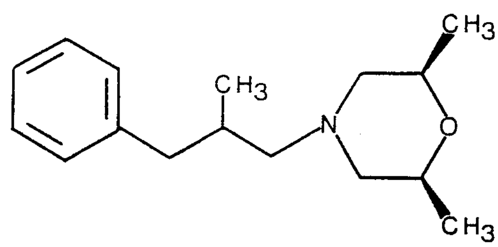

V

mit einer Verbindung umsetzt, die ein Carboniumion der allgemeinen Formel

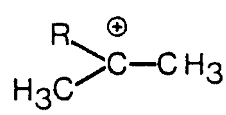

VI

worin R die oben angegebene Bedeutung besitzt, liefert, oder

d) eine Verbindung der Formel

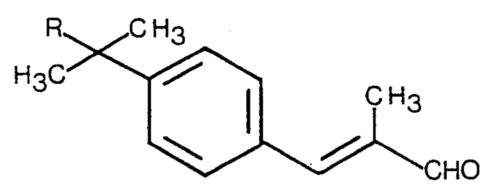

VII

worin R die obige Bedeutung hat, unter hydrierenden Bedingungen mit einer Verbindung der Formel III umsetzt, oder dass man, zwecks Herstellung eines N-Oxyds, eine Verbindung der Formel I mit Wasserstoffperoxid oder Persäuren behandelt, oder dass man zwecks Herstellung eines Salzes eine Base der Formel I mit einer Säure in an sich bekannter Weise in ein Salz überführt.

6. Verfahren nach Anspruch 5, dadurch gekennzeichnet, dass man eine Verbindung der Formel IV,

worin sich die Doppelbindung in α-Stellung zum Morpholinrest befindet, katalytisch oder mit Ameisensäure reduziert.

7. Verfahren nach Anspruch 5, dadurch gekennzeichnet, dass man eine Verbindung der Formel IV, worin sich die Doppelbindung in α-Stellung zum Phenylrest befindet, katalytisch reduziert.

8. Verfahren nach Anspruch 5, 6 oder 7, dadurch gekennzeichnet, dass man ein erhaltenes Racemat in die optischen Antipoden aufspaltet.

9. Verfahren zur Herstellung eines pharmazeutischen Präparats, dadurch gekennzeichnet, dass man mindestens eine der in den Ansprüchen 1 bis 3 genannten Verbindungen mit zur therapeutischen Verabreichung geeigneten, nicht-toxischen, inerten, an sich in solchen Präparaten üblichen festen und/oder flüssigen Trägern mischt und in eine geeignete Form bringt.

10. Pharmazeutisches Präparat, enthaltend mindestens eine der in den Ansprüchen 1 bis 3 genannten Verbindungen und nicht-toxisches, inertes Trägermaterial.

11. Pharmazeutisches Präparat gemäss Anspruch 10 zur Bekämpfung von Infektionen, hervorgerufen durch pathogene Pilze oder Hefen.

**Patentansprüche für den Vertragsstaat:** AT

1. Verfahren zur Herstellung von Verbindungen der Formel

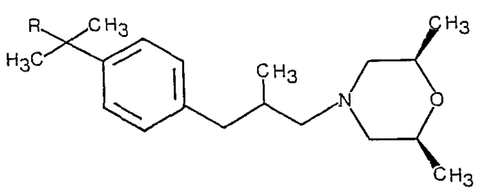

I

worin R Äthyl oder Phenyl bedeutet, sowie von Salzen und N-Oxyden hiervon, dadurch gekennzeichnet, dass man

a) ein Halogenid der Formel

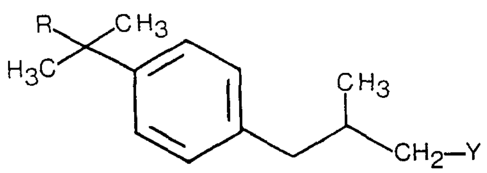

II

worin R die oben angegebene Bedeutung besitzt und Y Chlor, Brom oder Jod bedeutet, mit einer Verbindung der Formel

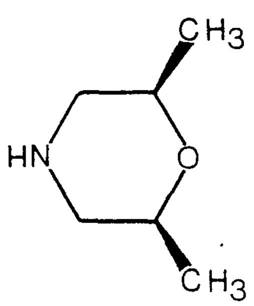

III

umsetzt, oder

b) eine Verbindung der Formel

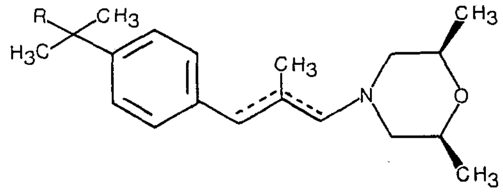

IV

worin R die obige Bedeutung hat und eine der beiden gestrichelten Linien eine weitere Bindung darstellt, reduziert, oder

c) eine Verbindung der allgemeinen Formel

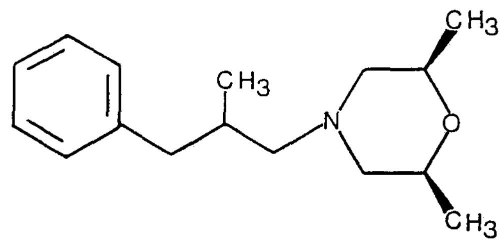

V

mit einer Verbindung umsetzt, die ein Carboniumion der allgemeinen Formel

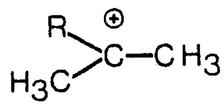

VI

worin R die oben angegebene Bedeutung besitzt, liefert, oder

d) eine Verbindung der Formel

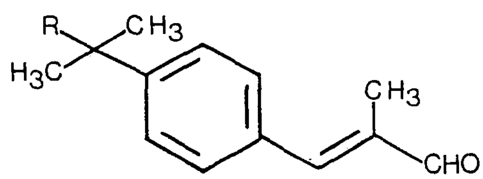

VII

worin R die obige Bedeutung hat, unter hydrierenden Bedingungen mit einer Verbindung der Formel III umsetzt, oder dass man, zwecks Herstellung eines N-Oxyds, eine Verbindung der Formel I mit Wasserstoffperoxid oder Persäuren behandelt, oder dass man zwecks Herstellung eines Salzes eine Base der Formel I mit einer Säure in an sich bekannter Weise in ein Salz überführt.

2. Verfahren nach Anspruch 1, dadurch gekennzeichnet, dass man eine Verbindung der Formel IV, worin sich die Doppelbindung in α-Stellung zum Morpholinrest befindet, katalytisch oder mit Ameisensäure reduziert.

3. Verfahren nach Anspruch 1, dadurch gekennzeichnet, dass man eine Verbindung der Formel IV, worin sich die Doppelbindung in α-Stellung zum Phenylrest befindet, katalytisch reduziert.

4. Verfahren nach Anspruch 1, dadurch gekennzeichnet, dass man ein erhaltenes Racemat in die optischen Antipoden aufspaltet.

5. Verfahren nach Anspruch 1, dadurch gekennzeichnet, dass man eine Verbindung der Formel VII

unter hydrierenden Bedingungen mit einer Verbindung der Formel III umsetzt.

6. Verfahren zur Herstellung eines pharmazeutischen Präparats, dadurch gekennzeichnet, dass man mindestens eine Verbindung der Formel I, oder ein Salz oder N-Oxyd hiervon mit zur therapeutischen Verabreichung geeigneten, nicht-toxischen, inerten, an sich in solchen Präparaten üblichen festen und/ oder flüssigen Trägern mischt und in eine geeignete Form bringt.

**Claims for the Contracting States:**
BE, CH, DE, FR, GB, IT, LI, LU, NL, SE

1. Compounds of the general formula

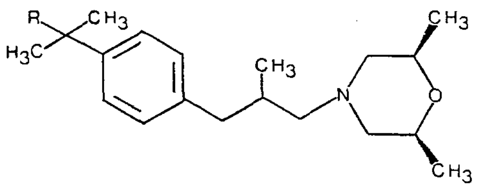

I

wherein R signifies ethyl or phenyl, and salts as well as N-oxides of these compounds.

2. Cis-4-[3-(p-tert.-amyl-phenyl)-2-methyl-propyl]-2,6-dimethyl-morpholine and its salts, as well as its N-oxide.

3. Cis-4-/3-[p-(α,α-dimethyl-benzyl)-phenyl-2--methyl-propyl/-2,6-dimethyl-morpholine and its salts, as well as its N-oxide.

4. Compounds according to any one of claims 1 to 3 as antimycotics.

5. Process for the manufacture of compounds of the general formula

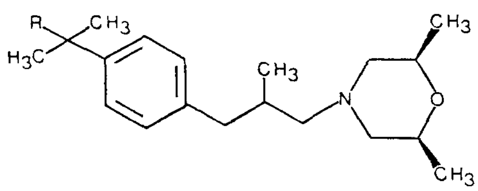

I

wherein R signifies ethyl or phenyl, and of salts and N-oxides thereof, characterized by

a) reacting a halide of the formula

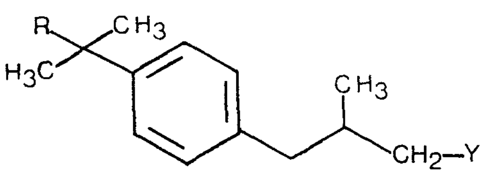

II

wherein R has the significance given above and Y signifies chlorine, bromide or iodine, with a compound of the formula

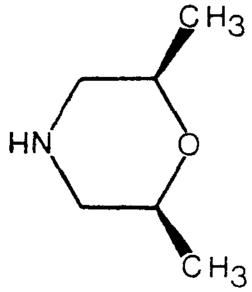

III

or

b) reducing a compound of the formula

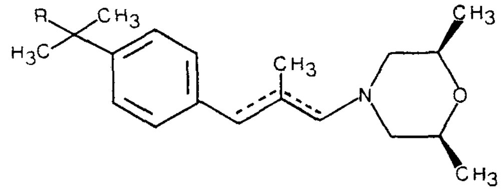

IV

wherein R has the above significance and one of the two dotted lines represents an additional bond, or

c) reacting a compound of the general formula

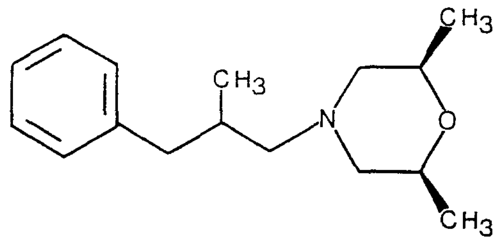

V

with a compound which yields a carbonium ion of the general formula

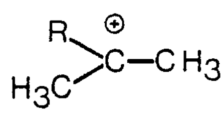

VI

wherein R has the significance given above, or

d) reacting a compound of the formula

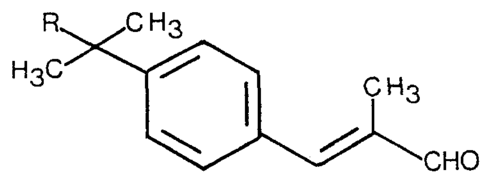

VII

wherein R has the above significance, under hydrogenating conditions with a compound of formula III, or, for the purpose of manufacturing a N-oxide, treating a compound of formula I with hydrogen peroxide or peracids, or, for the purpose of manufacturing a salt, converting a base of formula I into a salt with an acid in a manner known per se.

6. Process according to claim 5, characterized in that a compound of formula IV wherein the double bond is situated in the α-position to the morpholine residue is reduced catalytically or with formic acid.

7. Process according to claim 5, characterized in that a compound of formula IV wherein the double bond is situated in the α-position to the phenyl residue is reduced catalytically.

8. Process according to claim 5, 6 or 7, characterized in that a racemate obtained is resolved into the optical antipodes.

9. Process for the manufacture of a pharmaceutical preparation, characterized by mixing at least one of the compounds set forth in claims 1 to 3 with non-toxic, inert, solid and/or liquid carriers customary in such preparations and suitable for therapeutic administration and bringing the mixture into a suitable form.

10. Pharmaceutical preparation, containing at

least one of the compounds set forth in claims 1 to 3 and non-toxic, inert carrier material.

11. Pharmaceutical preparation in accordance with claim 10 for combatting infections caused by pathogenic fungi or yeasts.

**Claims for Contracting State:** AT

1. Process for the manufacture of compounds of the formula

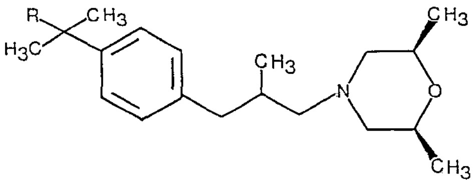
I

wherein R signifies ethyl or phenyl,
as well as of salts and N-oxides thereof, characterized by

a) reacting a halide of the formula

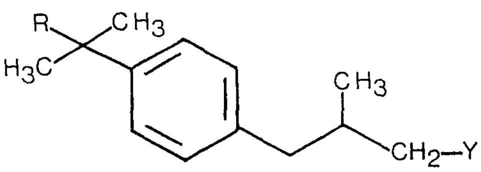
II

wherein R has the significance given above and Y signifies chlorine, bromine or iodine,
with a compound of the formula

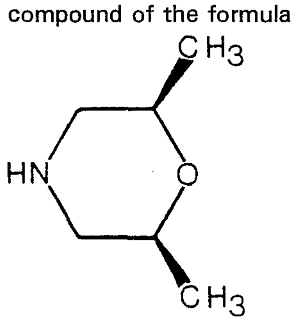
III

or

b) reducing a compound of the formula

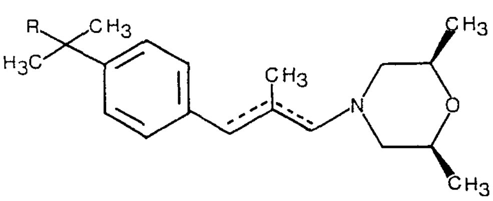
IV

wherein R has the above significance and one of the two dotted lines represents an additional bond, or

c) reacting a compound of the general formula

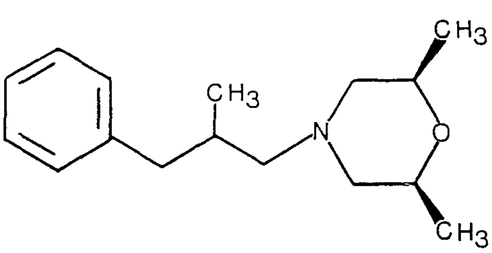
V

with a compound which yields a carbonium ion of the general formula

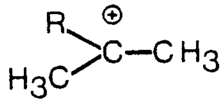
VI

wherein R has the significance given above, or

d) reacting a compound of the formula

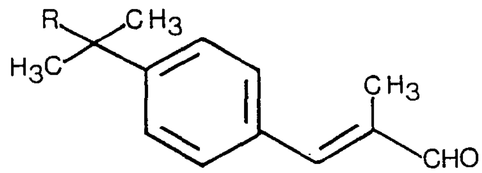
VII

wherein R has the above significance,
under hydrogenating conditions with a compound of formula III, or, for the purpose of manufacturing a N-oxide, treating a compound of formula I with hydrogen peroxide or peracids, or, for the purpose of manufacturing a salt, converting a base of formula I into a salt with an acid in a manner known per se.

2. Process according to claim 1, characterized in that a compound of formula IV wherein the double bond is situated in the α-position to the morpholine residue is reduced catalytically or with formic acid.

3. Process according to claim 1, characterized in that a compound of formula IV wherein the double bond is situated in the α-position to the phenyl residue is reduced catalytically.

4. Process according to claim 1, characterized in that a racemate obtained is resolved into the optical antipodes.

5. Process according to claim 1, characterized in that a compound of formula VII is reacted under hydrogenating conditions with a compound of formula III.

6. Process for the manufacture of a pharmaceutical preparation, characterized by mixing at least one compound of formula I or a salt or N-oxide thereof with non-toxic, inert, solid and/or liquid carriers customary in such preparations and suitable for therapeutic administration and bringing the mixture into a suitable form.

**Revendications pour les Etats contractants:**
BE, CH, DE, FR, GB, IT, LI, LU, NL, SE

1. Composés de formule générale

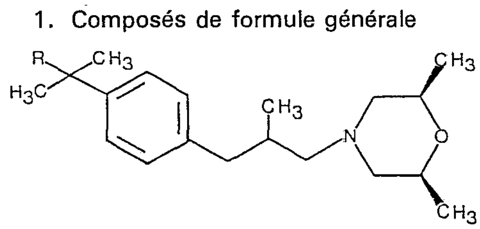
I

dans laquelle R représente un groupe éthyle ou phényle,
et les sels et N-oxydes de ces composés.

2. La cis-4-[3-(p-tert-amyl-phényl)-2-méthyl-propyl]-2,6-diméthyl-morpholine, ses sels et son N-oxyde.

3. La cis-4-/3-[p-(α,α-diméthyl-benzyl)-phé-

nyl]-2-méthyl-propyl/-2,6-diméthyl-morpholine, ses sels et son N-oxyde.

4. Composés selon l'une des revendications 1 à 3 en tant qu'antimycotiques.

5. Procédé de préparation des composés de formule générale

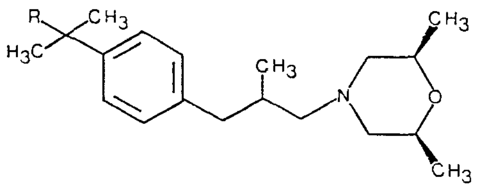
I

dans laquelle R représente un groupe éthyle ou phényle,

et de leurs sels et N-oxydes, caractérisé en ce que

a) on fait réagir un halogénure de formule

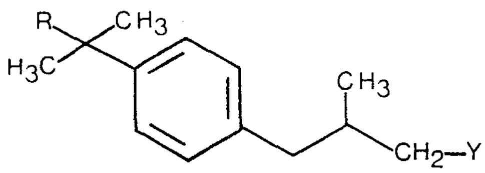
II

dans laquelle R a la signification indiquée ci-dessus et Y représente le chlore, le brome ou l'iode, avec un composé de formule

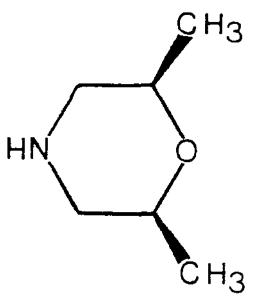
III

ou bien

b) on réduit un composé de formule

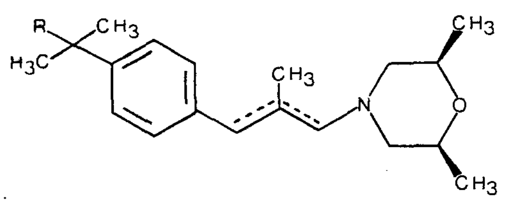
IV

dans laquelle R a la signification indiquée ci-dessus et l'un des deux traits interrompus représente une autre liaison, ou bien

c) on fait réagir un composé de formule générale

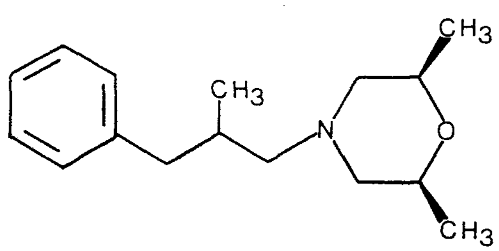
V

avec un composé fournissant un ion carbonium de formule générale

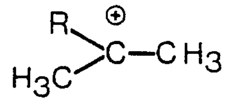
VI

dans laquelle R a la signification indiquée ci-dessus, ou bien

d) on fait réagir un composé de formule

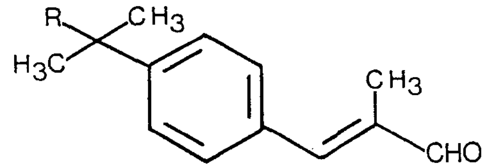
VII

dans laquelle R a la signification indiquée ci-dessus, dans des conditions hydrogénantes avec un composé de formule III ou bien, pour la préparation d'un N-oxyde, on traite un composé de formule I par le peroxyde d'hydrogène ou des peracides ou bien, pour la préparation d'un sel d'une base de formule I et d'un acide, on convertit en un sel de manière connue en soi.

6. Un procédé selon la revendication 5, caractérisé en ce que l'on soumet un composé de formule IV dans laquelle la double liaison est en position alpha du reste de morpholine, à réduction catalytique ou par l'acide formique.

7. Un procédé selon la revendication 5, caractérisé en ce que l'on soumet un composé de formule IV dans laquelle la double liaison est en position alpha du reste phényle, à réduction catalytique.

8. Procédé selon la revendication 5, 6 ou 7, caractérisé en ce que l'on résout un racémat obtenu en les antipodes optiques.

9. Procédé de préparation d'une composition pharmaceutique, caractérisé en ce que l'on mélange au moins un des composés mentionnés dans les revendications 1 à 3 avec des véhicules solides et/ou liquides, non toxiques, inertes, appropriés à l'administration thérapeutique, usuels dans ce type de composition, et on met sous une forme appropriée.

10. Composition pharmaceutique contenant au moins un des composés mentionnés dans les revendications 1 à 3 et un véhicule non toxique inerte.

11. Composition pharmaceutique selon la revendication 10, pour combattre les infections provoquées par des mycètes ou des levures pathogènes.

**Revendications pour l'Etat:** AT

1. Procédé de préparation des composés de formule

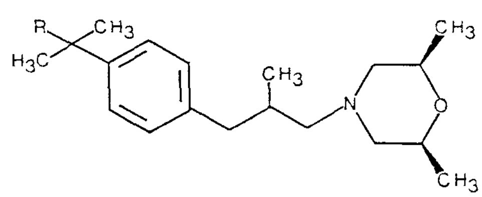
I

dans laquelle R représente un groupe éthyle ou phényle,

et de leurs sels et N-oxydes, caractérisé en ce que

a) on fait réagir un halogénure de formule

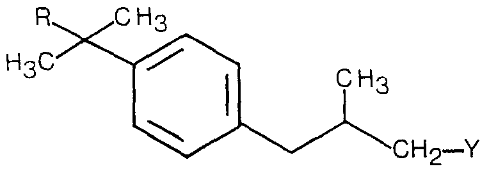

II

dans laquelle R a la signification indiquée ci-dessus et Y représente le chlore, le brome ou l'iode, avec un composé de formule

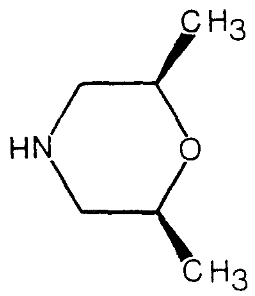

III

ou bien

b) on réduit un composé de formule

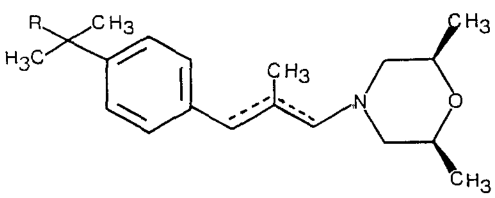

IV

dans laquelle R a la signification indiquée ci-dessus et l'un des deux traits interrompus représente une autre liaison, ou bien

c) on fait réagir un composé de formule générale

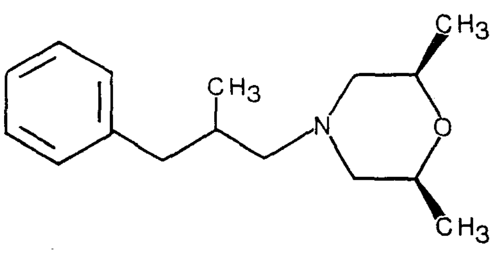

V

avec un composé fournissant un ion carbonium de formule générale

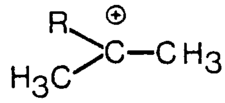

VI

dans laquelle R a la signification indiquée ci-dessus ou bien

d) on fait réagir un composé de formule

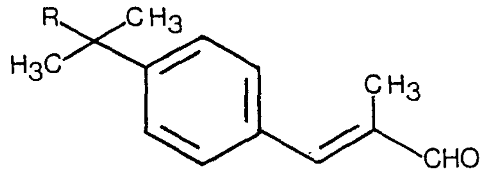

VII

dans laquelle R a la signification indiquée ci-dessus, dans des conditions hydrogénantes, avec un composé de formule III, ou bien, pour la préparation d'un N-oxyde, on traite un composé de formule I par le peroxyde d'hydrogène ou des peracides, ou bien, pour la préparation d'un sel d'une base de formule I et d'un acide, on convertit en un sel de manière connue en soi.

2. Procédé selon la revendication 1, caractérisé en ce que l'on soumet un composé de formule IV dans laquelle la double liaison est en position alpha du reste de morpholine, à réduction catalytique ou par l'acide formique.

3. Procédé selon la revendication 1, caractérisé en ce que l'on soumet un composé de formule IV dans la double liaison est en position alpha du reste phényle, à reduction catalytique.

4. Procédé selon la revendication 1, caractérisé en ce que l'on résout un racémate obtenu en les antipodes optiques.

5. Procédé selon la revendication 1, caractérisé en ce que l'on fait réagir un composé de formule VII dans des conditions hydrogénantes avec un composé de formule III.

6. Procédé de préparation d'une composition pharmaceutique, caractérisé en ce que l'on mélange au moins un composé de formule I ou un sel ou N-oxyde d'un tel composé avec des véhicules solides et/ou liquides non toxiques, inertes, appropriés à l'administration thérapeutique et usuels dans ce type de composition, et on met sous une forme appropriée.